# FASCICULE DE BREVET EUROPEEN

(11) **EP 3 841 978 B1**
(45) Date de publication et mention de la délivrance du brevet: **12.10.2022**
(21) Numéro de dépôt: 20211562.2
(22) Date de dépôt: 03.12.2020
(51) Int. Cl.: A61B 5/352, A61B 5/283, A61B 5/361

(54) **PROCÉDÉ ET DISPOSITIF POUR TRAITER UN SIGNAL CARDIAQUE**
VERFAHREN UND VORRICHTUNG ZUR VERARBEITUNG EINES HERZSIGNALS
METHOD AND DEVICE FOR PROCESSING A HEART RATE SIGNAL

(30) Priorité: 04.12.2019 FR 1913710
(43) Date de publication de la demande: 30.06.2021
(73) Titulaire: Sorin CRM SAS, 92140 Clamart (FR)
(72) Inventeur: VITALI, Luca, 10019 Strambino (IT); MARIE, Elodie, 14400 Vienne en Bessin (FR)
(74) Mandataire: Ungerer, Olaf

(56) Documents cités:
- EP-A1- 2 407 097
- US-A1- 2007 078 488

## Description

La présente invention concerne un dispositif et son procédé de traitement d'un signal provenant du cœur, en particulier un défibrillateur cardiaque implantable à sonde sous-cutanée.

La détection rapide et fiable des arythmies cardiaques est la clé du fonctionnement d'un défibrillateur. Des chocs retardés ou manqués peuvent être mortels pour un patient souffrant de fibrillation ventriculaire ainsi que pour d'autres types d'arythmie hémodynamiquement instable (c'est-à-dire dans laquelle le cœur ne produit pas de circulation sanguine). Dans le même temps, il convient d'éviter les chocs inutiles, car ils sont généralement associés à plusieurs effets indésirables.

Afin de détecter les événements arythmiques, les défibrillateurs examinent généralement les signaux électriques générés par le cœur et interprètent ces signaux afin de déterminer le rythme cardiaque. Lorsque le rythme cardiaque s'écarte de la normalité et remplit certains critères (par exemple, il est plus rapide qu'un rythme prédéterminé), une tachycardie est supposée détectée et un choc peut être délivré au patient.

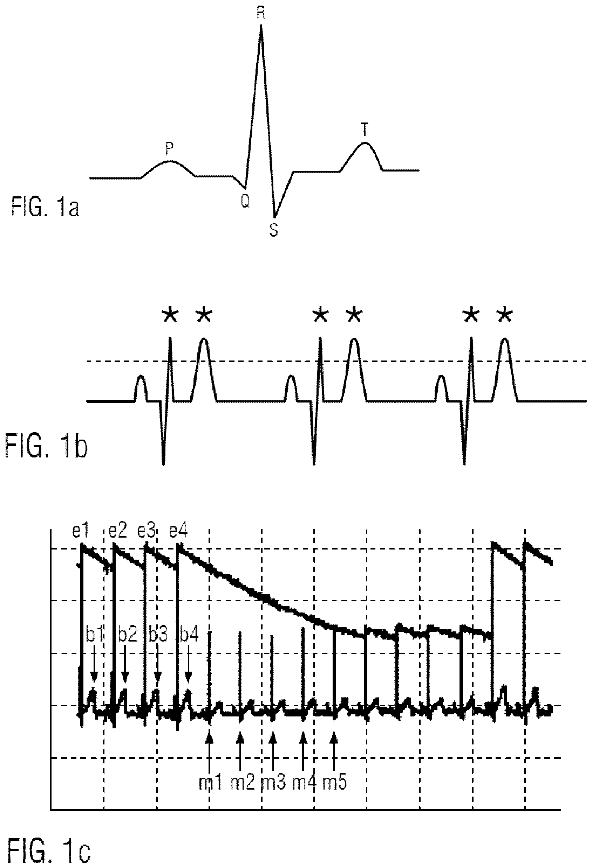

Un facteur clé pour détecter le rythme cardiaque est la capacité à différencier le complexe QRS, tel qu'illustré à la Figure 1a, qui marque le début de la contraction ventriculaire des autres composantes du signal cardiaque électrique, notamment l'onde T et l'onde P. Si le dispositif est capable de détecter tous les complexes QRS et seulement les complexes QRS, une détection de rythme fiable peut être effectuée.

Toutefois, détecter de manière fiable le rythme cardiaque s'avère plus complexe dans un défibrillateur sous-cutané que pour un défibrillateur classique endocavitaire. En effet, dans un défibrillateur endocavitaire, les signaux électriques tendent à présenter une morphologie plus favorable que dans un défibrillateur sous-cutané pour détecter le rythme cardiaque. Cela résulte, entre autre, du fait qu'avec une sonde implantée dans le ventricule droit, le composant le plus important du signal est le potentiel de dépolarisation, représentatif du complexe QRS illustré à la Figure 1a; alors que l'onde T et l'onde P sont pratiquement absentes ou peuvent être efficacement discriminées par un filtre passe-haut ou passe-bande approprié.

Dans un défibrillateur sous-cutané, les signaux cardiaques détectés par les électrodes situées sur la sonde sous-cutanée ressemblent davantage à ceux d'un électrocardiogramme (ECG) de surface standard dans lequel toutes les composantes sont présentes, parfois avec des caractéristiques morphologiques et d'amplitude qui rendent difficile une détection cohérente du rythme cardiaque.

Les méthodes de détection du rythme cardiaque couramment utilisées sont basées sur des étapes de traitement caractéristiques. Une première étape consiste généralement à amplifier et à filtrer le signal ECG d'entrée, dans le but d'améliorer les composantes souhaitées et de supprimer les composantes moins intéressantes. Cependant, un filtrage inadapté ou agressif peut conduire à la perte de composantes utiles à la détection du rythme cardiaque.

Typiquement, le signal prétraité est ensuite comparé à un seuil prédéterminé; lorsque le seuil est franchi, un cycle est compté. Le comptage de cycle est usuellement inhibé pendant une courte période, dite période réfractaire, après la détection d'un franchissement de seuil, pour éviter, par exemple, un comptage redondant d'un même cycle. Un exemple de comptage redondant de cycle est illustré à la Figure 1b. Ce défaut de comptage est souvent rencontré dans les défibrillateurs sous-cutanés et peut être provoqué, comme dans l'exemple illustré à la Figure 1b, par la détection incorrecte de l'onde T dans un cycle cardiaque (en plus de la détection du complexe QRS). Lorsque ce phénomène se produit, chaque cycle cardiaque est compté deux fois, avec pour conséquence un doublement apparent de la fréquence cardiaque, ce qui peut conduire à une fausse détection d'une tachycardie et à une délivrance inappropriée de chocs.

Tel qu'illustré à la Figure 1b, nombre de méthodes de détection du rythme cardiaque sont liées à la comparaison du signal prétraité (amplification, filtrage, etc.) avec un seul niveau de seuil. Une conséquence de cette approche à « seuil unique » est qu'il est essentiel que le seuil soit toujours correctement placé par rapport aux caractéristiques du signal, afin de pouvoir compter correctement les cycles cardiaques. En effet, un seuil placé trop haut, par exemple plus haut que les pics les plus élevés du signal, ne serait jamais franchi: dans ce cas, le système ne détecterait aucun cycle cardiaque, pourtant présent physiologiquement. On parle dans ce cas de « sous-détection », qui amène à une détection incorrecte du rythme cardiaque. Si, au contraire, le seuil est placé à un niveau trop bas, comme dans l'exemple de la Figure 1b, il peut être franchi non seulement par les pics du complexe QRS, mais également par d'autres composants (sur la Figure 1b, l'onde T), créant ainsi les conditions d'un comptage multiple de même cycle.

Pour pallier aux difficultés liées au positionnement adéquat du seuil, il est connu de faire varier le niveau de seuil selon un schéma basé, par exemple, sur l'amplitude des pics les plus élevés du signal, comme illustré à la Figure 1c. De cette manière, le seuil est assuré d'être aussi loin que possible du bruit et des complexes non désirés, qui sont normalement d'amplitude inférieure (voir les quatre complexes b1, b2, b3, b4 à la Figure 1c).

Il s'avère cependant que cette approche n'est pas assez robuste en ce qui concerne les changements d'amplitude rapides, comme illustré dans la Figure 1c où les cinq pics indiqués par les flèches m1, m2, m3, m4, m5 sont omis car ils sont masqués par les quatre pics précédent plus élevés (voir e1, e2, e3, e4 sur la Figure 1c) qui ont conduit à l'élévation du niveau seuil de détection, bien au-delà de l'amplitude des pics m1 à m5.

De plus, la mise en œuvre de procédés tels que décrits ci-dessous, pour filtrer le signal d'entrée et implémenter une méthode de détection par franchissement de seuil de détection, conduisent à des algorithmes complexes et coûteux en ressources logicielles. Les documents EP2407097A1 et US2007/078488A1 divulguent des procédés de traitement d'un signal cardiaque.

Dans le but de résoudre les limitations énumérées ci-dessus, la présente invention a pour objet d'améliorer la fiabilité et la robustesse du traitement d'un signal cardiaque, en particulier d'un signal recueilli par une sonde sous-cutanée, tout en minimisant les étapes de filtrage du signal en entrée le coût en ressources logicielles.

L'objet de la présente invention est atteint par un procédé de traitement d'un signal cardiaque représenté en fonction du temps comprenant les étapes de : a) fournir un nombre n de différents niveaux de seuil Ni, avec i=1 à n et n étant égal ou supérieur à trois ; b) détecter, à partir d'un temps T donné et par niveau de seuil Ni, au moins deux intersections successives du signal cardiaque avec le niveau de seuil Ni, en considérant un franchissement par valeur croissante et/ou décroissante du signal cardiaque avec le niveau de seuil Ni ; et c) déterminer au moins un paramètre statistique du signal cardiaque à partir des intersections du signal cardiaque avec les au moins trois différents niveaux de seuil Nᵢ.

Ainsi, au lieu de dépendre d'un seul seuil, fixe ou évolutif dans le temps comme décrit précédemment au regard des procédés connus, au moins trois différents niveaux de seuil espacés sont considérés en même temps par le présent procédé. Le signal est ainsi comparé simultanément à tous les seuils, ce qui permet d'obtenir un modèle de franchissement de seuil basé sur les intersections du signal cardiaque avec les différents niveaux de seuil. De plus, ce procédé ne requiert pas de discrimination par filtrage. En éliminant la discrimination par filtrage, toutes les informations présentes dans le signal sont préservées et peuvent être exploitées. Ceci est particulièrement avantageux lorsque les différences de morphologie, d'amplitude, de fréquence entre les composants désirés et ceux non-désirés deviennent subtiles et qu'il est important de préserver chaque élément d'information pour effectuer le traitement du signal cardiaque.

La présente invention, relative à un procédé de traitement d'un signal cardiaque, peut être davantage améliorée grâce aux modes de réalisation suivants.

Selon un mode de réalisation, la détermination du au moins un paramètre statistique à l'étape c) peut comprendre la détermination d'une période du signal cardiaque.

Ainsi, les différentes intersections du signal cardiaque avec les niveaux de seuils contribuent ensemble à la détermination d'une période du signal cardiaque, c'est-à-dire non seulement les pics du complexe QRS, mais aussi l'onde T ou même l'onde P, qui sont généralement filtrées autant que possible pour les procédés connus, afin qu'elles ne soient pas détectées.

Selon un mode de réalisation, l'étape b) peut en outre comprendre la détermination à partir du temps T donné et par niveau de seuil Ni, d'au moins une durée écoulée Δ₁tᵢ, i=1 à n, entre deux intersections successives du signal cardiaque avec le niveau de seuil Ni; et tel que le au moins un paramètre statistique peut être déterminé à l'étape c) au moyen des durées écoulées Δ₁tᵢ déterminées à l'étape b) à partir du temps T donné.

Ainsi, le procédé ne requiert pas de calculs complexes pour arriver à la détermination du paramètre statistique, ce qui permet de simplifier les calculs et de réduire les coûts de ressources logicielles.

Selon un mode de réalisation, une première durée écoulée Δ₁tᵢ peut être déterminée par niveau de seuil Ni entre les deux plus récentes intersections successives à partir du temps T donné ; et une deuxième durée écoulée Δ₂tᵢ peut être déterminée par niveau de seuil Ni entre l'intersection la plus récente et la troisième intersection la plus récente à partir du temps T donné ; l'étape c) comprenant en outre la détermination d'au moins un paramètre statistique du signal cardiaque en comparant les premières durées écoulées Δ₁tᵢ aux deuxièmes durées écoulées Δ₂tᵢ.

Ainsi, le procédé se caractérise davantage par une approche plutôt "stochastique" que "déterministe", comme les procédés antérieurs connus. Dans les autres procédés connus, des franchissements périodiques et précis de type "déterministe" avec un seuil unique sont nécessaires car chaque franchissement apporte l'information complète sur la fin d'un cycle cardiaque et le début d'un autre. Au contraire, dans le présent procédé selon l'invention, chaque intersection (c'est-à-dire le franchissement d'un des seuils par le signal) contribue à la détermination du paramètre statistique, aucune intersection n'étant plus importante que les autres. De plus, les caractéristiques des signaux cardiaques sont typiquement telles que pour la plupart des franchissements de seuils, la durée écoulée Δ₁tᵢ ou/et la durée écoulée Δ₂tᵢ est proche de la période que l'on cherche à déterminer, c'est-à-dire du rythme cardiaque.

Selon un mode de réalisation, à l'étape c), la période du signal cardiaque à partir du temps T donné peut être déterminée à partir de la répartition en nombre des durées écoulées Δ₁tᵢ; Δ₂tᵢ qui, en particulier, est représentée au moyen d'un histogramme.

Ainsi, le procédé, ne requiert pas de calculs complexes pour arriver à la détermination du paramètre statistique, ce qui permet de simplifier les calculs et de réduire les coûts de ressources logicielles, car dans la répartition, par exemple représenté par un histogramme, c'est la durée en temps, Δ₁tᵢ; ou en Δ₂tᵢ, qui a été le plus observée qui correspond à la valeur de la période du signal cardiaque, c'est-à-dire au rythme cardiaque.

En effet, des valeurs de durée similaires tendent à converger autour d'une valeur commune, s'accumulant et donnant lieu à une barre plus élevée pour cette durée dans l'histogramme. Il s'avère ainsi que la période du signal est bien lisible sur l'histogramme, même lorsque des composantes de faible amplitude sont présentes et sont prises en compte.

De plus, des changements dans la distribution des durées dans l'histogramme peuvent être l'indication d'un début d'une arythmie, car la distribution des valeurs de durée change rapidement et notamment le maximum diminue.

Selon un mode de réalisation, la répartition du nombre de durées écoulées (Δ1tᵢ ; Δ2tᵢ) par intervalle de temps défini peut être représentée au moyen d'un histogramme et la période du signal cardiaque à partir du temps T donné peut être déterminée à partir d'une moyenne ou d'une médiane des durées écoulées (Δ1tᵢ ; Δ2tᵢ) comprises dans la barre de l'histogramme qui comprend le plus grand nombre d'occurrence de durées écoulées (Δ1tᵢ ; Δ2tᵢ).

Ainsi, on peut déterminer de manière plus précise la durée de la période du signal cardiaque la plus fréquente à l'intérieur de l'intervalle de ladite barre de l'histogramme.

Selon un mode de réalisation, la répartition du nombre de durées écoulées (Δ1tᵢ ; Δ2tᵢ) par intervalle de temps défini peut être représentée au moyen d'un histogramme et dont la période du signal cardiaque à partir du temps T donné peut être déterminée à partir d'une moyenne ou d'une médiane des durées écoulées (Δ1tᵢ ; Δ2tᵢ) comprises dans un intervalle de temps défini plus large qu'un intervalle de temps correspondant à celui d'une barre de l'histogramme.

Ainsi, on peut déterminer de manière encore plus précise la durée de la période du signal cardiaque la plus fréquente en considérant les durées écoulées à la fois à l'intérieur de l'intervalle d'une barre de l'histogramme et au-delà de la barre de l'histogramme, délimité par l'intervalle défini.

Selon un mode de réalisation, à l'étape c) seules les durées écoulées (Δ₁tᵢ, ; Δ₂tᵢ) dont la valeur franchit un seuil prédéfini peuvent être prises en compte dans la détermination du au moins un paramètre statistique.

Ainsi, le seuil prédéfini permet de garantir la robustesse du procédé.

Selon un mode de réalisation, le seuil prédéfini peut être déterminé à partir de la détection d'intersections successives du signal cardiaque par niveau de seuil Ni, à un temps antérieur au temps T donné.

Ainsi, une étape de détection et de calcul supplémentaire n'est pas nécessaire pour déterminer le seuil prédéfini. Le présent procédé est ainsi davantage optimisé.

Selon un mode de réalisation, l'étape a) peut comprendre la détermination d'une amplitude minimale et d'une amplitude maximale du signal cardiaque; de sorte que les valeurs des différents niveaux de seuils Nᵢ peuvent être déterminées de manière à être comprises entre une valeur minimum et une valeur maximum correspondant respectivement à l'amplitude minimale et l'amplitude maximale dudit signal.

Ainsi, les différents niveaux de seuils Nᵢ sont déterminés de manière à assurer la robustesse du procédé.

Selon un mode de réalisation, la valeur de chacun des différents niveaux de seuils Ni peut être constante au cours du temps.

Ainsi, le présent procédé ne requiert pas la nécessité d'une adaptation continue des niveaux de seuils en fonction du temps, comme c'est le cas pour les procédés antérieurs connus. De ce fait, le coût de calcul informatique peut être réduit et le procédé est simplifié.

Selon un mode de réalisation, la valeur des différents niveaux de seuils Ni peut varier au cours du temps en fonction du au moins un paramètre statistique.

Ainsi, les différents niveaux de seuils Ni peuvent être adaptés de manière avantageuse selon le moment de la journée (phase de sommeil, phase active) ou/et au cours de la vie d'un patient.

Selon un mode de réalisation, les différents niveaux de seuils Ni peuvent être espacés par un écart fixe entre eux.

Ainsi, les différents niveaux de seuils Ni peuvent être avantageusement positionnés pour un signal cardiaque de manière à améliorer davantage la robustesse du procédé.

Selon un mode de réalisation, l'étape a) peut comprendre la détermination d'au moins dix différents niveaux de seuils Ni.

Comme chaque franchissement de seuil contribue dans le présent procédé, plus il y en a de seuils, meilleure est la détermination du paramètre statistique du signal cardiaque. Ainsi, la présence d'au moins dix différents niveaux de seuils Ni permet d'améliorer davantage la robustesse du procédé, notamment pour détecter les variations d'amplitude du signal.

Selon un mode de réalisation, chacun des niveaux de seuils Ni peuvent être différents d'une ligne de base.

La ligne de base peut être franchie plusieurs fois en fonction du temps, même sans signal, en raison de la présence de bruit intrinsèque dans chaque système. Le franchissement du seuil du niveau zéro, c'est-à-dire de la ligne de base, peut être affecté par du bruit que le signal soit absent ou présent. De ce fait, la ligne de base est avantageusement omise des différents niveaux de seuils Ni du procédé selon la présente invention.

Selon un mode de réalisation, le procédé peut en outre comprendre une étape de réception d'au moins un signal cardiaque par une sonde implantable d'un dispositif médical actif implantable qui est configurée pour recueillir un signal cardiaque par voie sous-cutanée.

Ainsi, le procédé est configuré pour traiter un signal cardiaque recueilli par voie sous-cutanée. Le procédé est en effet adapté pour traiter un tel signal cardiaque dont toutes les composantes sont présentes - complexe QRS, onde P et onde T. De ce fait, le procédé est configuré pour la détermination des caractéristiques morphologiques et d'amplitude d'un signal cardiaque recueilli par voie sous-cutanée.

Selon un mode de réalisation, le procédé peut en outre comprendre une étape de détermination d'un intervalle entre des franchissements de deux niveaux de seuils successifs Nᵢ=n et Nᵢ=n+1 par le signal cardiaque.

Ainsi, on obtient une valeur semblable à une dérivée du signal cardiaque qui permet de distinguer deux événements de même intensité mais de morphologie différente, tel qu'un pic du complexe QRS d'un artefact.

L'objet de la présente invention est également atteint par un dispositif médical actif implantable sous cutané, comprenant un boîtier ; une sonde implantable sous-cutanée connectée au boîtier ; la sonde implantable sous-cutanée comprenant une ou plusieurs électrodes de détection configurées pour recueillir des signaux cardiaques par voie sous-cutanée ; le dispositif comprenant en outre un circuit de contrôle configuré pour la mise en œuvre du procédé de traitement d'un signal cardiaque à partir d'au moins un des signaux cardiaques recueillis par la sonde sous-cutanée.

L'invention et ses avantages seront expliqués plus en détail dans la suite au moyen de modes de réalisation préférés et en s'appuyant notamment sur les figures d'accompagnement suivantes, dans lesquelles :
La Figure 1a représente un tracé standard d'un complexe QRS ;
La Figure 1b représente un signal cardiaque et un seuil constant selon une méthode connue de l'art antérieur ;
La Figure 1c représente un signal cardiaque et un seuil variable selon une méthode connue de l'art antérieure;
La Figure 2 représente un signal cardiaque et une pluralité de seuils selon le procédé de la présente invention ;
La Figure 3 représente un signal cardiaque et une pluralité de durées écoulées selon le procédé de la présente invention ;
La Figure 4a représente un signal cardiaque et une pluralité de durées écoulées selon un exemple du procédé de la présente invention ;
La Figure 4b représente un premier histogramme comprenant la pluralité de durées écoulées déterminées à partir du signal cardiaque de la Figure 4a du procédé de la présente invention ;
La Figure 4c représente un deuxième histogramme comprenant la pluralité de durées écoulées déterminées à partir du signal cardiaque de la Figure 4a du procédé de la présente invention ;
La Figure 5a représente un troisième histogramme comprenant une pluralité de durées écoulées déterminées à partir d'un signal cardiaque selon le procédé de la présente invention La Figure 5b représente un agrandissement du troisième histogramme illustré la Figure 5a ;
La Figure 6 représente deux graphiques déterminés pour discriminer un artefact du signal cardiaque selon la présente invention ;
La Figure 7a représente un signal cardiaque et des histogrammes correspondant en rythme sinusal normal ;
La Figure 7b représente un signal cardiaque et des histogrammes correspondant à l'approche d'un épisode de fibrillation ventriculaire ;
La Figure 7c représente un signal cardiaque et des histogrammes correspondant moins de deux secondes avant le premier cycle d'un épisode de fibrillation ventriculaire ;
La Figure 7d représente un signal cardiaque et des histogrammes correspondant pendant un épisode de fibrillation ventriculaire.

L'invention va maintenant être décrite plus en détail en utilisant des modes de réalisation avantageux d'une manière exemplaire et en référence aux dessins. Les modes de réalisation décrits sont simplement des configurations possibles et il faut garder à l'esprit que les caractéristiques individuelles telles que décrites ci-dessus peuvent être fournies indépendamment les unes des autres ou peuvent être omises tout à fait lors de la mise en œuvre de la présente invention.

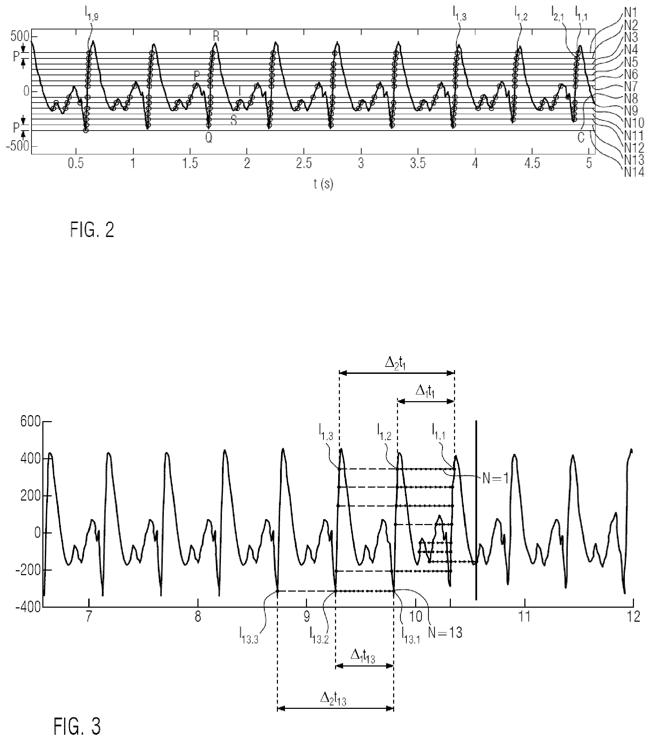

La courbe C de la Figure 2 représente un signal cardiaque d'un électrocardiogramme (ECG) en fonction du temps.

Les droites horizontales, parallèles à l'axe des abscisses, c'est-à-dire à l'axe temporel, représentent une pluralité de niveaux de seuil Nᵢ, avec i=1 à 14 dans l'exemple de la Figure 2. Selon la présente invention, le nombre de niveaux de seuil Ni peut varier tout en restant supérieur ou égal à 3. On notera que plus il y a de niveaux de seuil Ni, meilleure sera la détermination d'un paramètre statistique du signal cardiaque. Ainsi, considérer davantage de niveaux de seuil Ni permet d'améliorer la robustesse du procédé, notamment pour détecter les variations d'amplitude du signal cardiaque.

Chacun des cercles sur la Figure 2 représente le franchissement d'un certain niveau de seuil Ni par le signal cardiaque dans le sens ascendant, c'est-à-dire par valeur croissante du signal cardiaque. Ainsi, chaque cercle correspond à un point d'intersection I_{i,j} entre le signal cardiaque et un niveau de seuil Ni, avec i=1 à 14 et j=1 à 9 dans l'exemple de la Figure 2.

Dans une variante, le franchissement de chaque niveau de seuil par le signal cardiaque peut être considéré en sens descendant, c'est-à-dire par valeur décroissante du signal cardiaque.

Dans une autre variante, le franchissement des niveaux de seuil Ni par le signal cardiaque dans les deux sens (ascendant et descendant) peut être envisagé simultanément.

Dans l'exemple illustré par la Figure 2, le niveau de seuil correspondant au niveau zéro est volontairement omis car il correspond à la ligne de base qui peut être franchie plusieurs fois en fonction du temps, même sans signal, en raison du bruit intrinsèque dans chaque système physique. Ainsi, comme le franchissement du seuil de niveau zéro peut être affecté par du bruit que le signal soit présent ou absent, la pluralité des niveaux de seuil Ni, avec i=1 à 14 se distinguent volontairement du niveau zéro.

Dans l'exemple illustré par la Figure 2, la valeur de chacun des différents niveaux de seuils Ni est constante au cours du temps. Ainsi, le présent procédé ne requiert pas la nécessité d'une adaptation continue des niveaux de seuils en fonction du temps, comme c'est le cas pour des procédés antérieurs connus. De ce fait, le coût de calcul informatique peut être réduit et le procédé est simplifié.

Dans une variante, la valeur des différents niveaux de seuils Ni peut varier au cours du temps. Ainsi, les différents niveaux de seuils Ni peuvent être adaptés de manière avantageuse selon le moment de la journée (phase de sommeil, phase active) ou/et au cours de la vie d'un patient.

Selon l'exemple illustré par la Figure 2, les niveaux de seuil Ni avec i=1 à 7 et Ni avec i=8 à 14 sont espacés successivement des uns des autres par un même pas « p ». On notera que l'écart entre les niveaux de seuils Ni, c'est-à-dire la valeur du pas « p », peut être fixe ou évoluer au cours du temps.

Les différents niveaux de seuils Ni peuvent ainsi être avantageusement positionnés par rapport au signal cardiaque de manière à améliorer davantage la robustesse du procédé. En outre, le procédé de la présente invention peut comprendre la détermination d'une amplitude minimale et d'une amplitude maximale du signal cardiaque; de sorte que les valeurs des différents niveaux de seuils Ni sont sélectionnées de manière à être comprises entre une valeur minimum et une valeur maximum correspondant respectivement à l'amplitude minimale et l'amplitude maximale dudit signal cardiaque.

Ainsi, on assure qu'au cours du temps le signal cardiaque franchisse toujours au moins une pluralité de seuils Ni. Il peut néanmoins être accepté qu'au cours du temps le signal cardiaque ne franchisse pas la totalité des seuils Ni.

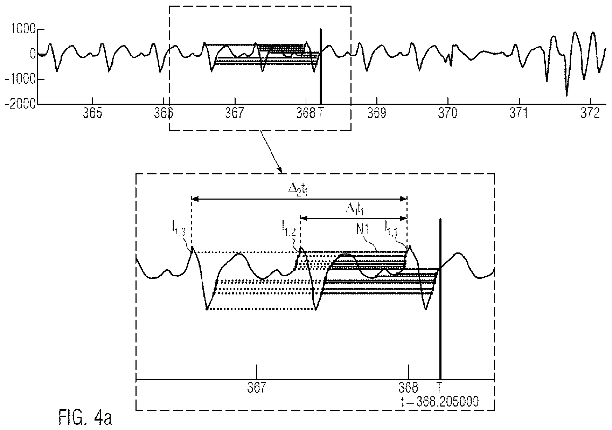

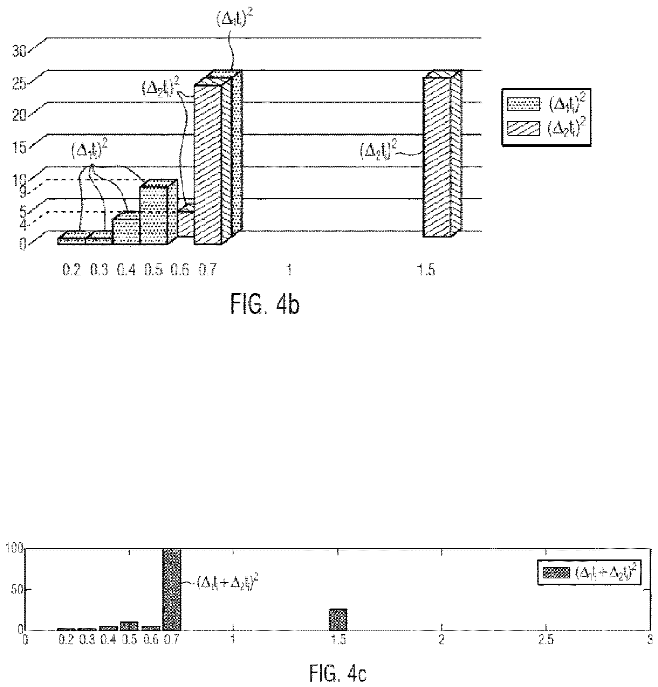

Selon la présente invention, le signal cardiaque est comparé simultanément à tous les seuils Ni ce qui permet d'obtenir un modèle statistique de franchissement de seuil. Un exemple de ce procédé est illustré à la Figure 3 et aux Figures 4a, 4b et 4c.

Le procédé selon la présente invention consiste à fournir un nombre n de différents niveaux de seuil Ni, avec i=1 à n et n étant égal ou supérieur à trois soit au moins trois différents niveaux de seuil Ni, tel qu'expliqué en référence à la Figure 2, et à détecter, à partir d'un temps T donné et par niveau de seuil Ni, au moins deux intersections I_{i,j} successives du signal cardiaque avec le niveau de seuil Ni, en considérant un franchissement par valeur croissante et/ou décroissante du signal cardiaque avec le niveau de seuil Ni.

Pour chaque niveau de seuil Ni, avec i=1 à n, une première durée écoulée Δ₁tᵢ est déterminée entre deux intersections successives I_{i,j} et I_{i,j+1} du signal cardiaque avec le niveau de seuil Nᵢ. La première durée écoulée Δ₁tᵢ correspond à la différence entre le franchissement le plus récent à partir du temps T et le deuxième franchissement le plus récent avec un même niveau de seuil. Sur la Figure 3, le segment Δ₁t₁ représente la première durée écoulée à partir du temps T pour le niveau de seuil N₁ entre la première intersection I_{1,1} et la deuxième intersection I_{1,2} du seuil N₁ avec le signal. De même, le segment Δ₁t₁₃ représente la première durée écoulée à partir du temps T pour le niveau de seuil N₁₃ entre la première intersection I_{13,1} et la deuxième intersection I_{13,2} du seuil N₁₃ avec le signal.

En outre, pour chaque niveau de seuil Ni, avec i=1 à n, une deuxième durée écoulée Δ₂tᵢ est déterminée par niveau de seuil Ni entre l'intersection la plus récente I_{i,j} et la troisième intersection I_{i,j+2} la plus récente à partir du temps T donné. La deuxième durée écoulée Δ₂tᵢ correspond à la différence entre le franchissement le plus récent à partir du temps T et le troisième franchissement le plus récent pour un même niveau de seuil. Sur la Figure 3, le segment Δ₂t₁ représente la deuxième durée écoulée à partir du temps T pour le niveau de seuil N₁ entre la première intersection I_{1,1} et la troisième intersection I_{1,3} du seuil N₁ avec le signal. De même, le segment Δ₂t₁₃ représente la deuxième durée écoulée à partir du temps T pour le niveau de seuil N₁₃ entre la première intersection I_{13,1} et la troisième intersection I_{13,3} du seuil N₁₃ avec le signal.

La détermination d'au moins un paramètre statistique du signal cardiaque est obtenue en comparant les premières durées écoulées Δ₁tᵢ aux deuxièmes durées écoulées Δ₂tᵢ en considérant la pluralité de niveaux de seuil Ni de manière simultanée.

On notera qu'une première durée Δ₁tᵢ et une deuxième durée Δ₂tᵢ sont déterminées pour chaque niveau de seuil Ni bien qu'elles ne soient pas indiquées sur la Figure 3 par souci de clarté.

On notera en outre que le niveau de seuil N₁₄ (illustré à la Figure 2), correspondant au seuil le plus bas, a volontairement été omis de la représentation de la Figure 3 car le signal cardiaque ne franchit pas le seuil N₁₄ dans la fenêtre de temps illustrée par la Figure 3. Il s'avère que le présent procédé n'exige pas que le signal franchisse tous les seuils Ni ; ce qui rend le procédé davantage tolérant aux variations d'amplitude du signal en comparaison aux méthodes connues basées sur un seul seuil. De manière avantageuse, le présent procédé tolère à la fois des variations lentes et soudaines de l'amplitude du signal cardiaque, dans la mesure où le signal franchit un nombre minimum de seuils, c'est-à-dire au moins trois, et en particulier au moins dix, afin que l'on puisse extraire certaines informations du signal, comme décrit ci-après.

Les Figures 4a, 4b et 4c représentent des étapes successives du procédé de traitement d'un signal cardiaque selon la présente invention. Les éléments ayant la même référence déjà utilisée aux figures 2 à 3 ne seront pas décrits à nouveau en détail, mais il est fait référence à leur description ci-dessus.

La Figure 4a représente un signal cardiaque et des niveaux de seuil Ni qui franchissent le signal en une pluralité d'intersections I_{i,j}. Tel qu'expliqué en référence à la Figure 3, le procédé de la présente invention comprend une étape à laquelle sont déterminées des premières durées écoulées Δ₁tᵢ et des deuxièmes durées écoulées Δ₂tᵢ. Par souci de clarté, seules les durées écoulées Δ₁t_{1 et} Δ₂t₁ ont été représentées sur le premier niveau de seuil N₁ de la Figure 4a. On notera que la détermination des durées écoulées pour les autres niveaux de seuil Ni est effectuée de la même manière que celle décrite à la Figure 3 à laquelle il est fait référence, et ne sera pas à nouveau décrite en détail.

Afin d'extraire un paramètre statistique du signal cardiaque, tel que la période du signal cardiaque pour déterminer par exemple le rythme cardiaque à partir des informations fournies par l'ensemble des durées Δ₁tᵢ et Δ₂tᵢ, lesdites durées Δ₁tᵢ et Δ₂tᵢ sont représentées graphiquement au moyen d'un histogramme.

La Figure 4b représente un tel histogramme qui est représenté en trois dimensions, et dans lequel les barres remplies de motifs à pois correspondent à la répartition des nombres N_{1,i} observés au carré des premières durées écoulées Δ₁tᵢ et les barres remplies de motifs à traits obliques correspondent à la répartition des nombres N_{2,i} au carré des deuxièmes durées écoulées Δ₂tᵢ. L'abscisse de l'histogramme de la Figure 4b représente des intervalles de temps en seconde tandis que l'ordonnée de l'histogramme représente le nombre d'occurrence des durées écoulées Δ₁tᵢ et Δ₂tᵢ observées dans chacun des intervalles de temps.

On notera que les durées Δ₁tᵢ et Δ₂tᵢ qui n'ont pas été mises à jour depuis longtemps, en particulier au-delà d'une durée prédéterminée, par exemple en raison d'une réduction de l'amplitude du signal, ne sont pas prises en compte dans l'histogramme. Pour mettre davantage en évidence les durées écoulées qui apparaissent le plus souvent dans le signal cardiaque, le nombre d'occurrences des durées écoulées Δ₁tᵢ et Δ₂tᵢ est élevé au carré dans la représentation de l'histogramme.

En raison, en particulier de l'allure inhérente d'un électrocardiogramme, un maximum apparaît dans l'histogramme pour une valeur commune de durées écoulées Δ₁tᵢ et Δ₂tᵢ. L'intervalle de temps pour lequel la fréquence d'occurrence des durées écoulées Δ₁tᵢ et Δ₂tᵢ est la plus élevée (c'est-à-dire la barre la plus haute) peut être considéré comme étant la période du signal cardiaque que l'on cherche à déterminer. Dans l'exemple illustré à la Figure 4b, à l'intervalle de temps centré sur t=0,7s on observe une majorité d'occurrences de premières durées écoulées Δ₁tᵢ et de deuxièmes durées écoulées Δ₂tᵢ.

On notera qu'à l'intervalle de temps centré sur t=1,5s on observe seulement une occurrence de deuxièmes durées écoulées Δ₂tᵢ mais pas de premières durées écoulées Δ₁tᵢ.

A la Figure 4c, les fréquences d'occurrence des durées écoulées Δ₁tᵢ et Δ₂tᵢ ont été additionnées entre elles puis élevées au carré afin de rendre l'histogramme davantage lisible, en élevant encore plus la barre la plus élevée par rapport aux autres barres de l'histogramme. Cet aspect est davantage expliqué au moyen du tableau 1 ci-après.

Le tableau 1 ci-dessous représente la fréquence d'occurrence par intervalle de temps pour le signal cardiaque illustré à la Figure 4a.

**[Tableau1]**

| t | Δ₁tᵢ | (Δ₁tᵢ)² | Δ₂tᵢ | (Δ₂tᵢ)² | Δ₁tᵢ+Δ₂tᵢ | (Δ₁tᵢ+Δ₂tᵢ)² |
|---|---|---|---|---|---|---|
| 0,2 | 1 | 1 | 0 | 0 | 1 | 1 |
| 0,3 | 1 | 1 | 0 | 0 | 1 | 1 |
| 0,4 | 2 | 4 | 0 | 0 | 2 | 4 |
| 0,5 | 3 | 9 | 0 | 0 | 3 | 9 |
| 0,6 | 0 | 0 | 2 | 4 | 2 | 4 |
| 0,7 | 5 | 25 | 5 | 25 | 10 | 100 |
| 1,5 | 0 | 0 | 5 | 25 | 5 | 25 |

L'histogramme de la Figure 4b représente les valeurs (Δ₁tᵢ)² et (Δ₂tᵢ)² du tableau 1 tandis que l'histogramme de la Figure 4c représente les valeurs (Δ₁tᵢ+Δ₂tᵢ)² du tableau 1. La barre la plus élevée de l'histogramme de la Figure 4c correspondant ainsi à la valeur 100. Selon le présent procédé, il est ainsi déterminé de manière statistique que la période du signal cardiaque représenté par la Figure 4a correspond à la durée indiquée par l'intervalle de temps de l'histogramme qui est centré sur t=0,7s à partir du temps T donné.

Dans l'exemple de la Figure 4c, on peut observer que l'histogramme fait sortir la valeur convenable du rythme cardiaque malgré la présence de composantes de faibles amplitudes qui sont prises en compte sans pour autant fausser le résultat, car pour les seuils franchis par les composantes de faibles amplitudes, la valeur Δ₂tᵢ indique la valeur convenable du rythme cardiaque. De ce fait, bien que des composantes de faibles amplitudes existent, elles ne changent pas pour autant l'interprétation du rythme cardiaque. Le présent procédé est ainsi davantage tolérant aux artefacts que les méthodes antérieures connues.

Notons que chaque intervalle de temps de l'histogramme correspond à une plage de valeur de temps et est centré sur une valeur de temps qui est celle indiquée en abscisse sur les Figures 4b et 4c. Ainsi, par exemple, l'intervalle de temps centré sur la valeur t=0,7s de la Figure 4c correspond à une plage de valeurs de temps comprises entre t=0,65s et t=0,75s.

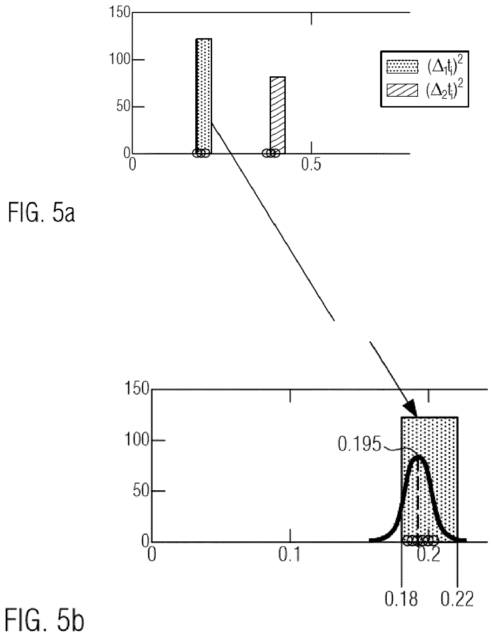

Selon une variante, on peut déterminer plus précisément la durée de la période du signal cardiaque la plus fréquente à l'intérieur d'une plage donnée. La Figure 5a illustre un histogramme avec une pluralité de barres de durées écoulées déterminées à partir d'un signal cardiaque selon le procédé de la présente invention décrit ci-dessus. La Figure 5b est une vue agrandie de la barre la plus haute de l'histogramme représentée à la Figure 5a. Elle correspond à un intervalle compris entre les valeurs de temps t=0,18s et t=0,22s et qui est centré sur la valeur t=0,20. La Figure 5b illustre également la distribution des durées écoulées à l'intérieur de cette plage t= [0,18 ; 0,22]. Chacune des valeurs en temps respectives des durées écoulées est indiquée par un cercle à la Figure 5b. A partir de cette distribution, une valeur moyenne des durées écoulées peut être déterminée. Dans une variante, c'est une médiane des durées écoulées qui peut être déterminée.

Dans l'exemple illustré par la Figure 5b, la moyenne des durées écoulées Δ₁tᵢ dans l'intervalle de temps t= [0,18 ; 0,22] centré sur la valeur t=0,20s a pour valeur t=0,195s.

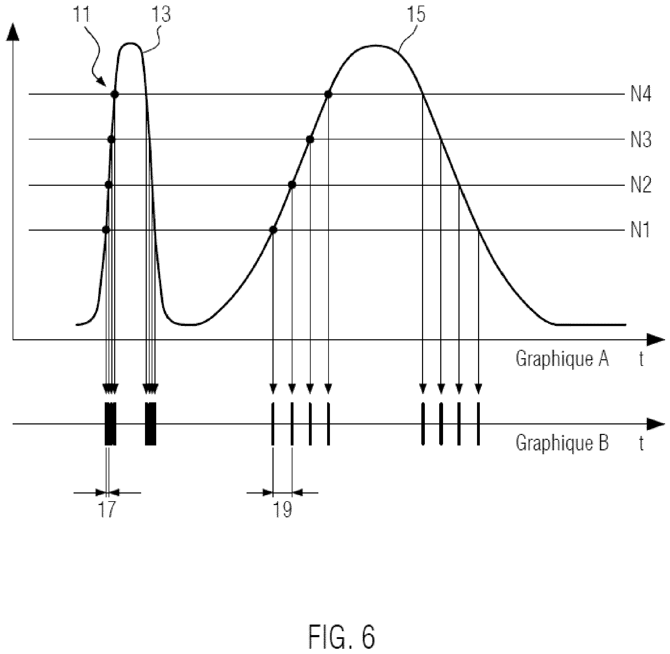

Dans une variante, la moyenne des durées écoulées est déterminée sur une plage de valeurs plus large qu'un intervalle de temps défini par la largeur d'une barre de l'histogramme. Le franchissement des seuils Nᵢ par le signal cardiaque peut également être utilisé pour déterminer un intervalle entre les franchissements de seuil de deux seuils successifs Nᵢ₌ₙ et Nᵢ₌ₙ₊₁. Ainsi, on obtient une valeur semblable à une dérivée du signal cardiaque qui permet de distinguer deux événements de même intensité mais de morphologie différente, comme expliqué dans ce qui suit en référence à la Figure 6 illustrant des graphiques A et B.

Le graphique A représente un signal cardiaque 11, en particulier un signal cardiaque 11 redressé, comprenant deux pics indiqués par les numéros de référence 13 et 15. Le graphique A illustre aussi quatre niveaux de seuils N₁, N₂, N₃, N₄.

Le graphique B représente le temps (indiqué par chaque trait vertical sur le graphique B) auquel le signal cardiaque 11 franchit l'un des niveaux de seuils N₁ à N₄.

Un intervalle de temps 17 est défini entre les franchissements successifs du pic 13 avec le niveau de seuil N₁ et le niveau de seuil suivant, c'est-à-dire le niveau de seuil N₂. De même, un intervalle de temps 19 est défini entre les franchissements du pic 15 avec le niveau de seuil N₁ et le niveau de seuil N₂. De la même manière, une pluralité d'intervalles de temps est définie pour les franchissements des pics 13 et 15 avec le reste des niveaux de seuil Ni.

Tel qu'illustré par le graphique B de la Figure 6, les intervalles de temps entre deux franchissements de niveaux de seuils relatifs au pic 13 sont plus courts que les intervalles de temps entre deux franchissements de niveaux de seuils relatifs au pic 15. On observe que l'intervalle de temps 17 est inférieur à l'intervalle de temps 19.

A partir des informations fournies par les intervalles de temps 17, 19 tels qu'illustrés au graphique B, il devient ainsi possible de distinguer deux différents types de pics du signal cardiaque, notamment de différencier un pic relatif au rythme cardiaque (comme le pic 13 du graphique A) d'un pic généré par un artefact (comme le pic 15 du graphique A).

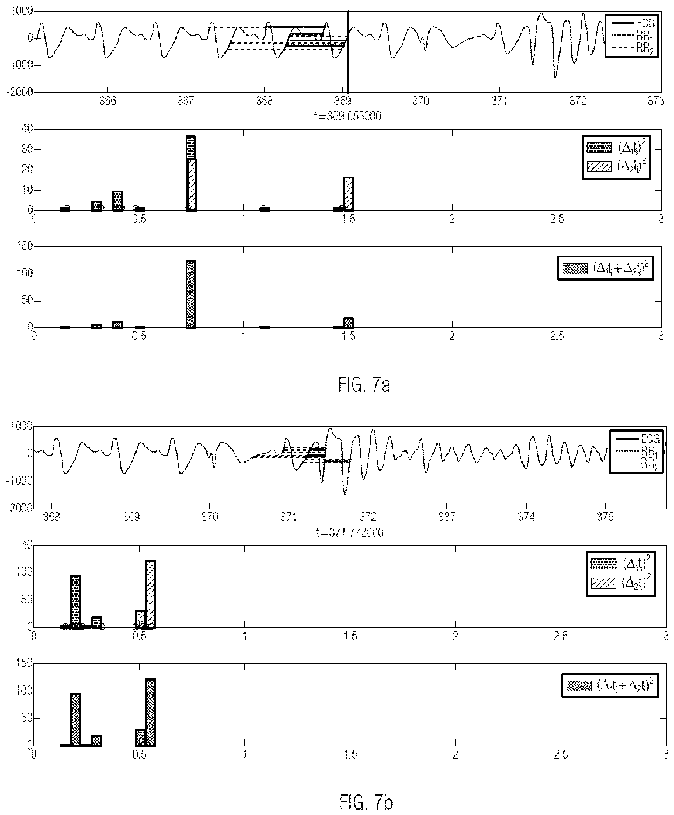

En outre, grâce à la considération simultanée d'une pluralité de niveaux de seuil Ni, le présent procédé est adapté à la détection d'un rythme cardiaque lors d'une fibrillation ventriculaire car le procédé de traitement s'avère davantage robuste et fiable et moins dépendant d'événements particuliers, comme des artefacts par exemple. Cet aspect est davantage décrit et illustré dans ce qui suit au moyen des Figures 7a à 7d.

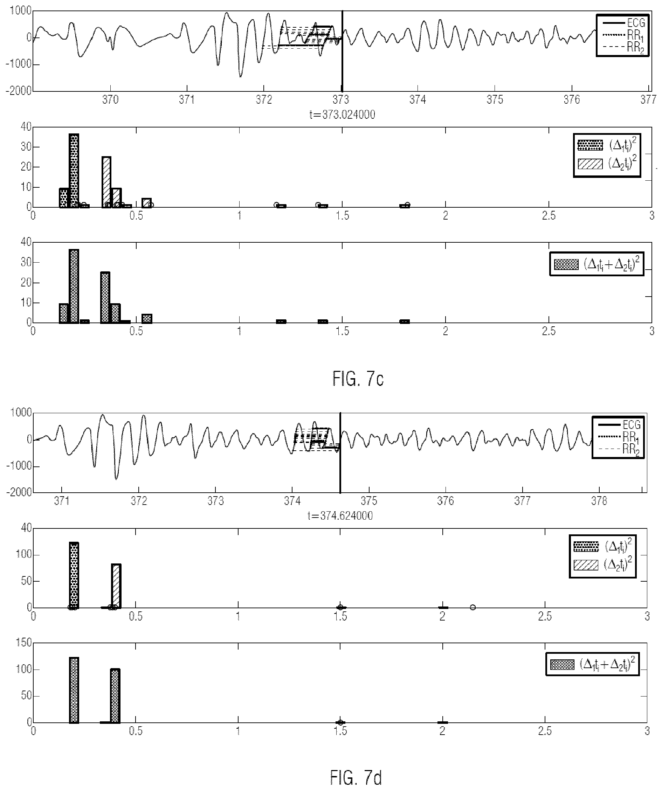

Les Figures 7a à 7d illustrent la manière dont le présent procédé réagit lors de la transition d'un rythme sinusal normal vers un épisode de fibrillation ventriculaire (aussi abrégée « FV » dans ce qui suit). Chacune des Figures 7 représente un signal cardiaque et deux histogrammes correspondants, déterminés de la même manière qu'aux Figures 4b et 4c.

La Figure 7a illustre un cycle en rythme sinusal normal dont le présent procédé permet de déterminer que la durée prédominante de la période du cycle cardiaque, telle qu'indiquée par l'histogramme, correspond à la barre la plus haute dont l'intervalle est centré sur la valeur t=0,7s. A l'approche d'une fibrillation ventriculaire, une accélération du rythme cardiaque est déjà perceptible au moyen du présent procédé. En effet, tel que représenté par la Figure 7b, dès l'entrée dans une période FV l'histogramme commence à être davantage dispersé, indiquant une « turbulence » dans le rythme cardiaque. Néanmoins, le rythme cardiaque reste encore relativement lent, avec une durée prédominante représentée sur l'histogramme de la Figure 7b à t=0,55s.

Une fibrillation ventriculaire démarre généralement comme une salve de tachycardie ventriculaire rapide et régulière qui se fragmente ensuite en de multiples ondelettes. La réduction soudaine de l'amplitude du signal cardiaque juste avant et/ou dès que l'épisode VF commence est un phénomène typique couramment observé. Cependant, avec les approches classiques des méthodes connues, il s'avère qu'un certain nombre de cycles est souvent manqué avant que les algorithmes de l'état de l'art ne parviennent à s'adapter à la nouvelle amplitude du signal et ne soient à nouveau capables de détecter le rythme cardiaque correctement.

L'histogramme de la Figure 7c est davantage dispersé que celui tracé en rythme sinusal normal (en comparaison à la Figure 7a). La dispersion des barres de l'histogramme de la Figure 7c indique une turbulence du rythme cardiaque. On observe de plus un décalage vers la gauche des barres de l'histogramme, c'est-à-dire une accélération du rythme cardiaque. Le présent procédé permet ainsi de détecter une accélération du rythme cardiaque juste avant que l'épisode VF ne commence, c'est-à-dire avant un premier cycle cardiaque en VF. La barre la plus haute de l'histogramme de la Figure 7a n'est presque plus visible à la Figure 7c.

Grâce à la présence de plusieurs niveaux de seuils selon l'invention et contrairement aux procédés connus de l'état de l'art, le présent procédé permet de suivre le signal cardiaque à l'approche de l'épisode VF même si l'amplitude baisse d'une manière significative. En effet, la Figure 7c illustre la détection d'un changement d'amplitude du signal sur un intervalle de temps d'environ une seconde, entre t=372s et t=373s, précédant l'épisode VF, au moyen de la pluralité des niveaux de seuils du présent procédé.

Ainsi, le présent procédé permet d'améliorer la détection d'une transition vers un épisode FV par rapport aux algorithmes connus.

La Figure 7d illustre un signal cardiaque durant un épisode de fibrillation ventriculaire. On observe par la morphologie du signal en forme d'ondelettes que l'amplitude du signal est réduite pendant l'épisode FV en comparaison avec l'amplitude du signal cardiaque précédant l'épisode VF, comme illustré à la Figue 7d par l'amplitude du signal avant et après t=373. L'histogramme de la Figure 7d met en évidence une répartition moins dispersée que celle illustrée à la Figure 7c, mais comprend des intervalles de temps avec des valeurs inférieures à ceux de la Figure 7a. En effet, la barre la plus élevée de la Figure 7d est centrée sur la valeur t=0,2s.

Ainsi, avec le présent procédé, il est possible d'observer une transition du rythme cardiaque d'un rythme cardiaque normal vers un épisode de VF au moyen des histogrammes, notamment en observant la dispersion et le déplacement des barres constituant les histogrammes.

Selon un mode de réalisation, le procédé peut en outre comprendre une étape de réception d'au moins un signal cardiaque par une sonde implantable d'un dispositif médical actif implantable qui est configurée pour recueillir un signal cardiaque par voie sous-cutanée.

Ainsi, le procédé est configuré pour traiter un signal cardiaque recueilli par voie sous-cutanée. Le procédé est en effet adapté pour traiter un tel signal cardiaque dont toutes les composantes sont présentes, c'est-à-dire le complexe QRS, l'onde P et l'onde T. De ce fait, le procédé est configuré pour la détermination des caractéristiques morphologiques et d'amplitude d'un signal cardiaque recueilli par voie sous-cutanée.

Notons que dans le cas particulier où l'histogramme, tel que ceux représentés aux Figures 7a à 7d, comprendrait deux barres de hauteurs sensiblement égales et distinctes l'une de l'autre, liées entre elles du fait que chacune des durées écoulées Δ₂tᵢ correspond au double des durées écoulées Δ₁tᵢ, le présent procédé est configuré pour sélectionner l'intervalle de temps de plus petite valeur, c'est-à-dire l'intervalle de temps correspondant aux durées écoulées Δ₁tᵢ pour la détermination du paramètre statistique.

Dans le cas où l'histogramme comprendrait deux barres juxtaposées entre elles et de hauteurs sensiblement égales, par exemple parce que certaines valeurs de durées écoulées se trouvaient à la frontière entre un intervalle et un autre, des analyses temporelles peuvent être effectuées en décalage d'une demi-période sur chaque barre de l'histogramme. Dans une variante, au lieu de représenter les durées écoulées au moyen d'un histogramme, une distribution temporelle des durées écoulées est considérée. Dans cette variante, les maximum d'une courbe de distribution temporelle des durées écoulées sont déterminés et utilisés pour la détermination du paramètre statistique.

La présente invention se rapporte également à un dispositif médical actif implantable sous cutané comprenant un boîtier, une sonde implantable sous-cutanée connectée au boîtier, la sonde implantable sous-cutanée comprenant une ou plusieurs électrodes de détection à partir desquels des signaux cardiaques sont recueillis par voie sous-cutanée. Ledit dispositif comprend en outre un circuit de contrôle configuré pour la mise en œuvre du procédé selon la présente invention à partir d'au moins un des signaux cardiaques recueillis par la sonde sous-cutanée, en particulier recueillis en temps réel. On notera que dans un défibrillateur sous-cutané, les signaux cardiaques détectés par la sonde sous-cutanée ressemblent davantage à ceux d'un électrocardiogramme (ECG) de surface standard dans lequel toutes les composantes (complexes QRS, ondes P et T) sont présentes, telles qu'illustrées sur les Figures 2, 3 et 4a.

Le circuit de contrôle du dispositif peut comprendre un microcontrôleur lui-même comprenant un processeur.

## Revendications

1. Un procédé de traitement d'un signal cardiaque représenté en fonction du temps comprenant les étapes de :
a) Fournir un nombre n de différents niveaux de seuil Ni, avec i=1 à n et n étant égal ou supérieur à trois ;
b) Détecter, à partir d'un temps T donné et par niveau de seuil Ni, au moins deux intersections successives du signal cardiaque avec le niveau de seuil Ni, en considérant un franchissement par valeur croissante et/ou décroissante du signal cardiaque avec le niveau de seuil Ni ;
c) Déterminer au moins un paramètre statistique du signal cardiaque à partir des intersections du signal cardiaque avec les au moins trois différents niveaux de seuil Ni.

2. Le procédé selon la revendication 1, dans lequel la détermination du au moins un paramètre statistique à l'étape c) comprend la détermination d'une période du signal cardiaque.

3. Le procédé selon la revendication 2, dans lequel l'étape b) comprend en outre la détermination à partir du temps T donné et par niveau de seuil Ni, d'au moins une durée écoulée Δ₁tᵢ, i=1 à n, entre deux intersections successives du signal cardiaque avec le niveau de seuil Ni; et tel que le au moins un paramètre statistique est déterminé à l'étape c) au moyen des durées écoulées Δ₁tᵢ déterminées à l'étape b) à partir du temps T donné.

4. Le procédé selon la revendication 3, dont une première durée écoulée Δ₁tᵢ est déterminée par niveau de seuil Ni entre les deux plus récentes intersections successives à partir du temps T donné ; et
une deuxième durée écoulée Δ₂tᵢ est déterminée par niveau de seuil Ni entre l'intersection la plus récente et la troisième intersection la plus récente à partir du temps T donné ;
l'étape c) comprenant en outre la détermination d'au moins un paramètre statistique du signal cardiaque en comparant les premières durées écoulées Δ₁tᵢ aux deuxièmes durées écoulées Δ₂tᵢ.

5. Le procédé selon la revendication 3 ou 4, dont à l'étape c), la période du signal cardiaque à partir du temps T donné est déterminée à partir de la répartition du nombre de durées écoulées (Δ₁tᵢ; Δ₂tᵢ), en particulier au moyen d'un histogramme.

6. Le procédé selon la revendication 5, dont la répartition du nombre de durées écoulées (Δ₁tᵢ ; Δ₂tᵢ) par intervalle de temps défini est représentée au moyen d'un histogramme et dont la période du signal cardiaque à partir du temps T donné est déterminée à partir d'une moyenne ou d'une médiane des durées écoulées (Δ₁tᵢ; Δ₂tᵢ) comprises dans la barre de l'histogramme qui comprend le plus grand nombre d'occurrence de durées écoulées (Δ₁tᵢ; Δ₂tᵢ).

7. Le procédé selon la revendication 5, dont la répartition du nombre de durées écoulées (Δ₁tᵢ ; Δ₂tᵢ) par intervalle de temps défini est représentée au moyen d'un histogramme et dont la période du signal cardiaque à partir du temps T donné est déterminée à partir d'une moyenne ou d'une médiane des durées écoulées (Δ₁tᵢ; Δ₂tᵢ) comprises dans un intervalle de temps défini plus large qu'un intervalle de temps correspondant à celui d'une barre de l'histogramme.

8. Le procédé selon l'une des revendications 3 à 7, dans lequel à l'étape c) seules les durées écoulées (Δ₁tᵢ; Δ₂tᵢ) dont la valeur franchit un seuil prédéfini sont prises en compte dans la détermination du au moins un paramètre statistique.

9. Le procédé selon la revendication 8, dans lequel le seuil prédéfini est déterminé à partir de la détection d'intersections successives du signal cardiaque par niveau de seuil Ni, à un temps antérieur au temps T donné.

10. Le procédé selon une des revendications précédentes, dans lequel l'étape a) comprend la détermination d'une amplitude minimale et d'une amplitude maximale du signal cardiaque; de sorte que les valeurs des différents niveaux de seuils Ni sont déterminées de manière à être comprises entre une valeur minimum et une valeur maximum correspondant respectivement à l'amplitude minimale et l'amplitude maximale dudit signal.

11. Le procédé selon une des revendications précédentes, dans lequel la valeur de chacun des différents niveaux de seuils Ni est constante au cours du temps.

12. Le procédé selon une des revendications 1 à 10, dans lequel la valeur des différents niveaux de seuils Ni varient au cours du temps en fonction du au moins un paramètre statistique.

13. Le procédé selon une des revendications précédentes, dans lequel les différents niveaux de seuils Ni sont espacés par un écart fixe entre eux.

14. Le procédé selon l'une des revendications précédentes, dans lequel l'étape a) comprend la détermination d'au moins dix différents niveaux de seuils Nᵢ.

15. Le procédé selon l'une des revendications précédentes, dans lequel chacun des niveaux de seuils Ni sont différents d'une ligne de base.

16. Le procédé selon l'une des revendications précédentes, comprenant en outre une étape de réception d'au moins un signal cardiaque recueilli par une sonde implantable d'un dispositif médical actif implantable qui est configurée pour recueillir un signal cardiaque par voie sous-cutanée.

17. Le procédé selon l'une des revendications précédentes, comprenant en outre une étape de détermination d'un intervalle entre des franchissements de deux niveaux de seuils successifs Nᵢ₌ₙ et Nᵢ₌ₙ₊₁ par le signal cardiaque.

18. Un dispositif médical actif implantable sous cutané, comprenant :
un boîtier;
une sonde implantable sous-cutanée connectée au boîtier ;
la sonde implantable sous-cutanée comprenant une ou plusieurs électrodes de détection configurées pour recueillir des signaux cardiaques par voie sous-cutanée;
le dispositif comprenant en outre un circuit de contrôle configuré pour la mise en œuvre du procédé selon une des revendication 1 à 17 à partir d'au moins un des signaux cardiaques recueillis par la sonde sous-cutanée.

## Patentansprüche

1. Verfahren zum Verarbeiten eines als Funktion der Zeit dargestellten Herzsignals, umfassend die Schritte:
a) Bereitstellen einer Anzahl n von unterschiedlichen Schwellenpegeln Ni, wobei i = 1 bis n und n gleich oder größer als drei ist;
b) Detektieren von mindestens zwei aufeinanderfolgenden Schnittpunkten des Herzsignals mit dem Schwellenpegel Ni ab einer gegebenen Zeit T und pro Schwellenpegel Ni, wobei ein Kreuzen durch steigenden und/oder fallenden Wert des Herzsignals mit dem Schwellenpegel Ni betrachtet wird;
c) Bestimmen mindestens eines statistischen Parameters des Herzsignals aus den Schnittpunkten des Herzsignals mit den mindestens drei unterschiedlichen Schwellenpegeln Ni.

2. Verfahren nach Anspruch 1, wobei das Bestimmen des mindestens einen statistischen Parameters in Schritt c) das Bestimmen einer Periode des Herzsignals umfasst.

3. Verfahren nach Anspruch 2, wobei der Schritt b) ferner die Bestimmung von mindestens einer verstrichenen Zeitdauer Δ₁tᵢ, i = 1 bis n, ab der gegebenen Zeit T und pro Schwellenpegel Ni zwischen zwei aufeinanderfolgenden Schnittpunkten des Herzsignals mit dem Schwellenpegel Ni umfasst; und wobei der mindestens eine statistische Parameter in Schritt c) anhand der in Schritt b) ermittelten verstrichenen Zeitdauer Δ₁tᵢ ab der gegebenen Zeit T bestimmt wird.

4. Verfahren nach Anspruch 3, wobei eine erste verstrichene Zeitdauer Δ₁tᵢ pro Schwellenpegel Ni zwischen den zwei jüngsten aufeinanderfolgenden Schnittpunkten ab der gegebenen Zeit T bestimmt wird; und
wobei eine zweite verstrichene Zeitdauer Δ₂tᵢ pro Schwellenpegel Ni zwischen dem jüngsten Schnittpunkt und dem drittjüngsten Schnittpunkt ab der gegebenen Zeit T bestimmt wird;
wobei Schritt c) ferner das Bestimmen mindestens eines statistischen Parameters des Herzsignals durch Vergleichen der ersten verstrichenen Zeitdauer Δ₁tᵢ mit der zweiten verstrichenen Zeitdauer Δ₂tᵢ umfasst.

5. Verfahren nach Anspruch 3 oder 4, wobei in Schritt c) die Periode des Herzsignals ab der gegebenen Zeit T aus der Verteilung der Anzahl der verstrichenen Zeitdauern (Δ₁tᵢ; Δ₂tᵢ) ermittelt wird, insbesondere mittels eines Histogramms.

6. Verfahren nach Anspruch 5, wobei die Verteilung der Anzahl der verstrichenen Zeitdauern (Δ₁tᵢ; Δ₂tᵢ) pro definiertem Zeitintervall mittels eines Histogramms dargestellt wird und wobei die Periode des Herzsignals ab der gegebenen Zeit T aus einem Durchschnitt oder einem Median derjenigen verstrichenen Zeitdauern (Δ₁tᵢ; Δ₂tᵢ) ermittelt wird, die in dem Balken des Histogramms mit der größten Häufigkeit verstrichener Zeiten (Δ₁tᵢ; Δ₂tᵢ) enthalten sind.

7. Verfahren nach Anspruch 5, wobei die Verteilung der Anzahl der verstrichenen Zeitdauern (Δ₁tᵢ; Δ₂tᵢ) pro definiertem Zeitintervall mittels eines Histogramms dargestellt wird und wobei die Periode des Herzsignals ab der gegebenen Zeit T aus einem Mittelwert oder einem Median derjenigen verstrichenen Zeitdauern (Δ₁tᵢ; Δ₂tᵢ) ermittelt wird, die in einem definierten Zeitintervall enthalten sind, das breiter ist als ein Zeitintervall, das dem eines Balkens des Histogramms entspricht.

8. Verfahren nach einem der Ansprüche 3 bis 7, wobei bei der Bestimmung des mindestens einen statistischen Parameters in Schritt c) nur diejenigen verstrichenen Zeitdauern (Δ₁tᵢ; Δ₂tᵢ) berücksichtigt werden, deren Wert eine vorgegebene Schwelle überschreitet.

9. Verfahren nach Anspruch 8, wobei die vordefinierte Schwelle aus der Erfassung aufeinanderfolgender Schnittpunkte des Herzsignals pro Schwellenpegel Ni zu einer Zeit vor der gegebenen Zeit T bestimmt wird.

10. Verfahren nach einem der vorhergehenden Ansprüche, wobei Schritt a) das Bestimmen einer minimalen Amplitude und einer maximalen Amplitude des Herzsignals umfasst; so dass die Werte der verschiedenen Schwellenpegel Ni so bestimmt werden, dass sie zwischen einem minimalen Wert und einem maximalen Wert liegen, die der minimalen Amplitude bzw. der maximalen Amplitude des Signals entsprechen.

11. Verfahren nach einem der vorhergehenden Ansprüche, wobei der Wert jedes der unterschiedlichen Schwellenpegel Ni über die Zeit konstant ist.

12. Verfahren nach einem der Ansprüche 1 bis 10, wobei der Wert der verschiedenen Schwellenwerte Ni in Abhängigkeit von mindestens einem statistischen Parameter zeitlich variiert.

13. Verfahren nach einem der vorhergehenden Ansprüche, wobei die unterschiedlichen Schwellenpegel Ni einen festen Abstand voneinander aufweisen.

14. Verfahren nach einem der vorhergehenden Ansprüche, wobei Schritt a) die Bestimmung von mindestens zehn unterschiedlichen Schwellenpegeln Ni umfasst.

15. Verfahren nach einem der vorhergehenden Ansprüche, wobei jeder der Schwellenpegel Ni von einer Grundlinie abweicht.

16. Verfahren nach einem der vorhergehenden Ansprüche, ferner umfassend einen Schritt des Empfangens mindestens eines Herzsignals, das von einer implantierbaren Sonde einer implantierbaren aktiven medizinischen Vorrichtung erfasst wird, die zum subkutanen Erfassen eines Herzsignals konfiguriert ist.

17. Verfahren nach einem der vorhergehenden Ansprüche, das ferner einen Schritt zum Bestimmen eines Intervalls zwischen Schnittpunkten zweier aufeinanderfolgender Schwellenpegel Nᵢ₌ₙ und Nᵢ₌ₙ₊₁ mit dem Herzsignal umfasst.

18. Subkutan implantierbares aktives medizinisches Gerät, umfassend:
ein Gehäuse;
eine mit dem Gehäuse verbundene, subkutane implantierbare Sonde;
wobei die subkutane implantierbare Sonde eine oder mehrere Erfassungselektroden umfasst, die zur Erfassung subkutaner Herzsignale konfiguriert sind;
wobei die Vorrichtung ferner eine Steuerschaltung umfasst, die für die Durchführung des Verfahrens nach einem der Ansprüche 1 bis 17 anhand mindestens eines der von der subkutanen Sonde erfassten Herzsignale konfiguriert ist.

## Claims

1. A method of processing a cardiac signal represented as a function of time, comprising the steps of:
a) providing a number n of different threshold levels Nᵢ, where i=1 to n and n is equal to or greater than three;
b) detecting, from a given time T and per threshold level N₁, at least two successive intersections of the cardiac signal with the threshold level Nᵢ, by considering a crossing by increasing and/or decreasing value of the cardiac signal with the threshold level Nᵢ;
c) determining at least one statistical parameter of the cardiac signal from the intersections of the cardiac signal with the at least three different threshold levels Nᵢ.

2. A method according to claim 1, wherein determining the at least one statistical parameter in step c) comprises determining a period of the cardiac signal.

3. A method according to claim 2, wherein step b) further comprises determining, from the given time T and per threshold level Nᵢ, at least one elapsed duration Δ₁tᵢ, where i=1 to n, between two successive intersections of the cardiac signal with the threshold level Nᵢ, and in such a way that the at least one statistical parameter is determined in step c) by means of the elapsed durations Δ₁t₁ determined in step b) from the given time T.

4. A method according to claim 3, wherein a first elapsed duration Δ₁t₁ is determined per threshold level Nᵢ between the most recent two successive intersections from the given time T; and
a second elapsed duration Δ₂tᵢ is determined per threshold level Nᵢ between the most recent intersection and the third most recent intersection from the given time T,
step c) further comprising determining at least one statistical parameter of the cardiac signal by comparing the first elapsed durations Δ₁t₁ to the second elapsed durations Δ₂tᵢ.

5. A method according to claim 3 or 4, wherein, in step c), the period of the cardiac signal from the given time T is determined from the distribution of the number of elapsed durations (Δ₁tᵢ; Δ₂tᵢ), in particular by means of a histogram.

6. A method according to claim 5, wherein the distribution of the number of elapsed durations (Δ₁tᵢ; Δ₂tᵢ) per defined time interval is represented by means of a histogram, and wherein the period of the cardiac signal from the given time T is determined from an average or a median of the elapsed durations (Δ₁tᵢ; Δ₂tᵢ) included in the bar of the histogram that includes the highest number of occurrences of elapsed durations (Δ₁tᵢ; Δ₂tᵢ).

7. A method according to claim 5, wherein the distribution of the number of elapsed durations (Δ₁tᵢ; Δ₂tᵢ) per defined time interval is represented by means of a histogram, and wherein the period of the cardiac signal from the given time T is determined from a mean or a median of the elapsed durations (Δ₁tᵢ; Δ₂tᵢ) included in a defined time interval wider than a time interval corresponding to that of a bar of the histogram.

8. A method according to any one of claims 3 to 7, wherein, in step c), only the elapsed durations (Δ₁tᵢ; Δ₂tᵢ) whose value crosses a predefined threshold are taken into account in determining the at least one statistical parameter.

9. A method according to claim 8, wherein the predefined threshold is determined from detecting successive intersections of the cardiac signal per threshold level Nᵢ at a time prior to the given time T.

10. A method according to any one of the preceding claims, wherein step a) comprises determining a minimum amplitude and a maximum amplitude of the cardiac signal in such a way that the values of the different threshold levels Nᵢ are determined so as to be between a minimum value and a maximum value respectively corresponding to the minimum amplitude and the maximum amplitude of said signal.

11. A method according to any one of the preceding claims, wherein the value of each of the different threshold levels Nᵢ is constant over time.

12. A method according to any one of claims 1 to 10, wherein the value of the different threshold levels Nᵢ varies over time as a function of the at least one statistical parameter.

13. A method according to any one of the preceding claims, wherein the different threshold levels Nᵢ are spaced apart by a fixed distance between them.

14. A method according to any one of the preceding claims, wherein step a) comprises determining at least ten different threshold levels Nᵢ.

15. A method according to any one of the preceding claims, wherein each of the threshold levels Nᵢ is different from a base line.

16. A method according to any one of the preceding claims, further comprising a step of receiving at least one cardiac signal collected by an implantable probe of an implantable active medical device which is configured to collect a cardiac signal subcutaneously.

17. A method according to any one of the preceding claims, further comprising a step of determining an interval between crossings of two successive threshold levels Nᵢ₌ₙ and Nᵢ₌ₙ₊₁ by the cardiac signal.

18. A subcutaneous implantable active medical device, comprising:
a housing;
a subcutaneous implantable probe connected to the housing,
the subcutaneous implantable probe comprising one or more sensing electrodes configured to collect cardiac signals subcutaneously,
the device further comprising a control circuit configured to implement a method according to any one of claims 1 to 17 from at least one of the cardiac signals collected by the subcutaneous probe.
